# EUROPEAN PATENT SPECIFICATION

(11) **EP 4 075 451 B1**
(45) Date of publication and mention of the grant of the patent: **18.12.2024**
(21) Application number: 20898513.5
(22) Date of filing: 04.11.2020
(51) Int. Cl.: H01B 1/22, A61B 5/263, A61B 5/265, A61B 5/268

(54) **METHOD FOR PRODUCING BIOELECTRODE**
VERFAHREN ZUR HERSTELLUNG EINER BIOELEKTRODE
PROCÉDÉ DE FABRICATION DE BIOÉLECTRODE

(30) Priority: 12.12.2019 JP 2019224772
(43) Date of publication of application: 19.10.2022
(73) Proprietor: NOK Corporation, Tokyo 105-8585 (JP)
(72) Inventor: FUTASHIMA, Ryo, Kanagawa 2510042 (JP)
(74) Representative: Global IP Europe Patentanwaltskanzlei
(86) International application number: PCT/JP2020/041198
(87) International publication number: WO 2021/117379

(56) References cited:
- WO-A1-2018/008688
- WO-A1-2018/230445
- WO-A1-2019/139164
- OCHIAI, MITSURU: ""Aerosol industrial use" Fumed silica", JOURNAL OF AEROSOL RESEARCH, vol. 5, no. 1, 30 November 1989 (1989-11-30), JP , pages 32 - 43, XP009535369, ISSN: 0912-2834, DOI: 10.11203/jar.5.32

## Description

### Technical Field

The present invention relates to a method for manufacturing a biological electrode, and relates, for example, to a method for manufacturing a biological electrode containing silver powder.

### Background Art

Conventionally, thin plates of highly conductive metals such as gold, silver, platinum and copper have been used as materials for biological electrode. The material for biological electrode made of these highly conductive metals has poor adhesion to skin and insufficient detection of an electrical signal from the skin. Therefore, when the material for biological electrode made of these highly conductive metals is used, it is necessary to apply gel, cream, paste, or the like to the skin in order to improve detection of electric signals from the skin. In this case, since the applied material used remains on hair or the like and causes discomfort, aftertreatment such as hair washing has been required.

Therefore, in recent years, there has been proposed a conductive silicone rubber electrode or the like in which conductive carbon particles are blended into a silicone rubber as an electrode which does not require application of cream, paste, or the like (see Patent Document 1). Further, there has also been proposed a technique for imparting stretchability to a wiring substrate which can be mounted on a human body or the like in which a substrate and a coating layer constituting the wiring substrate include an elastomer, and the elastomer includes silicone rubber (see Patent Document 2).

The Patent Document 3 discloses a bioelectrode including a conductive rubber electrode and a silver coating layer. The silver coating layer is provided on the conductive rubber electrode, contains a silicone rubber and silver particles, and contains modified silicone and, between the silver particles, ions for ionic conduction.

Since the biological electrode described in Patent Document 1 is an electrode using a rubber material, it can be used repeatedly, and since it has flexibility, it has good adhesion to the skin. Further, since it combines electronic conduction and ionic conduction, it has high conductivity and strain resistance, and since the surface in contact with the skin is conductive by silver, the contact impedance is low, and it is possible to stably measure even in measurement under dry conditions without using paste or the like.

### Citation List

### Patent Documents

[Patent Document 1] WO 2018/008688 A1
[Patent Document 2] JP-A-2017-117861
[Patent Document 3] WO 2019/139164 A1

### Summary of the Invention

### Problem to be solved by the invention

However, while the biological electrode described in Patent Document 1 can be repeatedly used because it is a rubber electrode, the repeated use causes strain and increases the resistance value of the rubber electrode. In addition, depending on the silver powder used for the rubber electrode, the cohesive force is strong, and it is difficult to uniformly disperse the silicone rubber in a liquid silicone rubber because it becomes lumpy state at the time of manufacturing. Therefore, for example, at the time of manufacturing biological electrode, there has been a problem that it is necessary to perform an operation such as sieving and blending silver powder to be used immediately before preparation of a material and thus, the manufacturing process becomes complicated.

The present invention has been made in view of such circumstances, and provides a manufacturing method capable of easily manufacturing a biological electrode in which an increase in resistance value over time due to strain is suppressed.

### Means for Solving the Problem

According to the present invention, there is provided a method for manufacturing a biological electrode shown below.
[1] A method for manufacturing a biological electrode, characterized by:
   a step of adding fumed silica to an aggregated silver powder and mixing to obtain a mixed silver powder in which the silver powder and the fumed silica are dispersed; and
   a step of forming a conductive rubber body containing a silicone rubber and the mixed silver powder.
[2] The method for manufacturing a biological electrode according to [1], wherein hydrophobic fumed silica is used as the fumed silica.
[3] The method for manufacturing a biological electrode according to [1] or [2], wherein 1 to 5 parts by mass of the fumed silica is added to 100 parts by mass of the aggregated silver powder.
[4] The method for manufacturing a biological electrode according to any one of [1] to [3], wherein in the step of forming the conductive rubber body, the mixed silver powder is added to a room temperature-curable liquid silicone rubber to prepare a silver powder-containing paste, and the obtained silver powder-containing paste is cured to form the conductive rubber body.
[5] The method for manufacturing a biological electrode according to any one of [1] to [4], wherein in the step of forming the conductive rubber body, the conductive rubber body having a layered shape is formed.
[6] The method for manufacturing a biological electrode according to any one of [1] to [4], wherein in the step of forming the conductive rubber body, the conductive rubber body having a brushed shape is formed.
[7] A rubber for a biological electrode, which includes at least a silicone rubber, an aggregated silver powder, and a fumed silica, wherein 1 to 5 parts by mass of the fumed silica is contained in 100 parts by mass of the aggregated silver powder.
[8] A rubber electrode made of the rubber for a biological electrode according to [7].

### Effect of the Invention

According to the method for manufacturing a biological electrode of the present invention, it is possible to conveniently manufacture a biological electrode in which an increase in resistance value over time due to strain is suppressed.

### BRIEF DESCRIPTION OF THE DRAWINGS

Fig. 1 is a cross-sectional schematic view showing an example of a biological electrode obtained by the method for manufacturing a biological electrode according to an embodiment of the present invention.
Fig. 2 is an explanatory drawing representing the use state of the biological electrode obtained by the method for manufacturing the biological electrode according to an embodiment of the present invention.
Fig. 3A is an explanatory drawing of a signal transmitting member of the biological electrode obtained by the method for manufacturing the biological electrode according to an embodiment of the present invention.
Fig. 3B is an explanatory drawing of another example of the signal transmitting member of the biological electrode obtained by the method for manufacturing the biological electrode according to an embodiment of the present invention.
Fig. 4 is a diagram showing another configuration example of the biological electrode obtained by the method for manufacturing the biological electrode according to an embodiment of the present invention.
Fig. 5 is a perspective view showing another example of the biological electrode obtained by the method for manufacturing the biological electrode according to an embodiment of the present invention.
Fig. 6 is a cross-sectional view showing another example of the biological electrode obtained by the method for manufacturing the biological electrode according to an embodiment of the present invention.
Fig. 7 is a top view showing another example of the biological electrode obtained by the method for manufacturing the biological electrode according to an embodiment of the present invention.
Fig. 8 is a partially side view of another example of the biological electrode obtained by the method for manufacturing the biological electrode according to an embodiment of the present invention.
Fig. 9 is a graph showing the results of a sieve passage rate test.
Fig. 10 is a graph showing the measured results of a volume resistivity of the biological electrodes (before applying the load) in Examples 1 and 2 and Comparative Example 1.
Fig. 11 is a graph showing the measured results of the volume resistivity of the biological electrodes (after applying the load) in Examples 1 and 2 and Comparative Example 1.
Fig. 12 is an explanatory drawing for explaining a method of measuring the volume resistivity of the biological electrode.

### Mode for carrying out the Invention

### <Biological electrode>

The biological electrode obtained by the method for manufacturing the biological electrode in the embodiment of the present invention has a conductive rubber body containing a silicone rubber and a silver powder. The biological electrode may further have a conductive substrate if needed. When the biological electrode has a conductive substrate, it has a conductive rubber body on the conductive substrate. Since the silver powder is uniformly dispersed in the conductive rubber body constituting the biological electrode, an increase in resistance value over time due to strain is suppressed. The shape of the conductive rubber body constituting the biological electrode is not particularly limited, and the conductive rubber body can have a desired shape, such as a layered shape, a brushed shape, or an uneven shape, depending on the type and the surface shape of the object to be measured. Such a biological electrode has good adhesion to the subject's body, is soft to the touch, does not cause discomfort even if it is in close contact for a long time, and can maintain stable contact with the subject's body. Further, even in a three-dimensional shape such as a brushed shape, it expresses high conductivity and it is possible to stably measure with little noise. Further, since it is not necessary to use a gel or the like, it can be stably measured even in measurement under dry conditions, and the method of use is also simple.

Hereinafter, the biological electrode obtained by the method for manufacturing in the embodiment of the present invention (hereinafter, also referred to as "the manufacturing method according to the present embodiment") will be described in detail by referring to the attached drawings. The present invention is not limited in any way by the following embodiments.

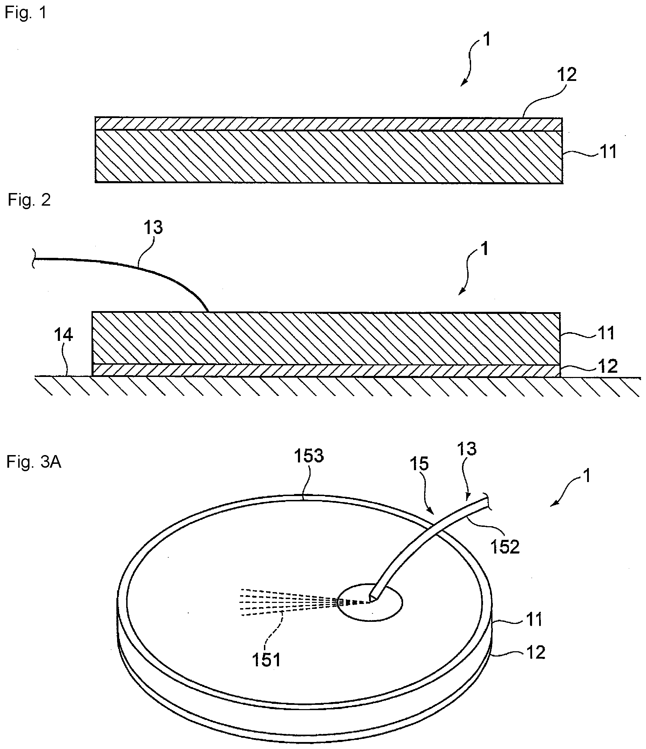

Fig. 1 is a cross-sectional schematic view showing an example of the biological electrode obtained by the manufacturing method according to the present embodiment. The biological electrode 1 according to the present embodiment is suitably used for at least one of sensing an electric signal from a living body and transmitting an electric stimulus to a living body. As shown in Fig. 1, the biological electrode 1 includes a conductive substrate 11 containing conductive silicone rubber, and a conductive rubber body 12 provided on the conductive substrate 11. In the example of Fig. 1, the conductive rubber body 12 has a layered shape. That is, in the biological electrode 1, the conductive rubber body 12 is provided as a conductive rubber layer covering the conductive substrate 11 containing conductive silicone rubber. Thus, adhesion of the conductive rubber body 12 to the living body can be further improved.

Fig. 2 is an explanatory drawing showing the use status of the biological electrode obtained by the manufacturing method of the biological electrode according to the present embodiment. As shown in Fig. 2, the biological electrode 1 contacts the surface of the conductive rubber body 12 and the living body 14 with, for example, a signal transmitting member 13 connected to a measuring instrument (not shown) connected to the surface of the conductive substrate 11. As a result, since the electric signal from the living body 14 is transmitted to the measuring instrument via the conductive rubber body 12, the conductive substrate 11, and the signal transmitting member 13, it is possible to measure the electric signal from the living body 14. Since the biological electrode 1 is used in direct contact with the living body, the conductive rubber body 12 has a predetermined contact area (area of the outermost surface). The contact area of the conductive rubber body 12 with the living body is preferably 0.2cm² or more and 13cm² or less, more preferably 1cm² or more and 8cm² or less, and still more preferably 3cm² or more and 7cm² or less. When the contact area of the conductive rubber body 12 is within the above range, the impedance can be lowered to effectively prevent the mixing of noise. In addition, the measured result is not affected by body movement, and the biological electrode 1 can be contacted even at a small site of the living body.

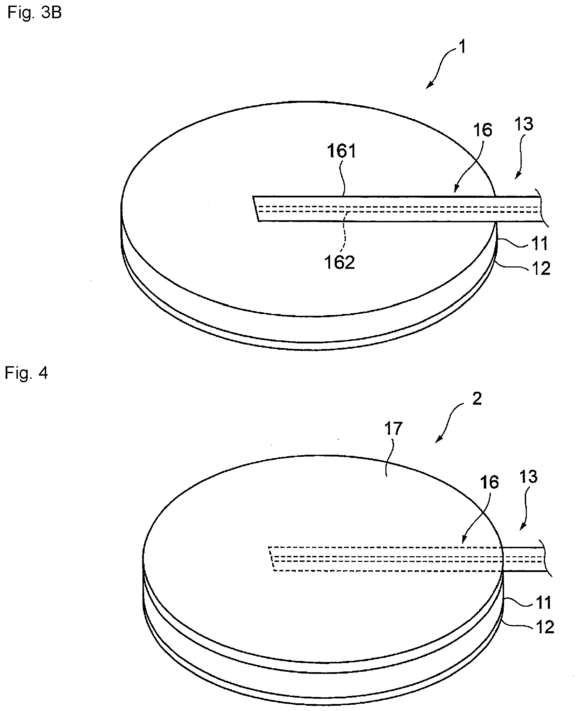

Figs. 3A and 3B are explanatory drawings of the signal transmitting member of the biological electrode obtained by the manufacturing method of the biological electrode according to the present embodiment. The example shown in Fig. 3A shows an example using a covering wire 15 as the signal transmitting member 13. The covering wire 15 includes a conductive metal core wire 151 and a resin covering material 152 covering the core wire 151. In the covering wire 15, the distal end portion of the core wire 151 is exposed from the covering material 152. The exposed distal end portion of the core wire 151 is fixed to the surface of the conductive substrate 11 by an adhesive tape 153 or the like. With such a configuration, since the electric signal flowing through the conductive substrate 11 is transmitted to the outside by the covering wire 15 via the core wire 151, it is possible to transmit the electric signal from the living body 14 to the outside.

The example shown in Fig. 3B shows an example using a flexible printed substrate 16 as the signal transmitting member 13. The flexible printed substrate 16 includes a resin base film 161 and a metal conductive foil 162 provided on the base film 161. The conductive foil 162 is a copper or a copper plated with gold. With such a configuration, since the electrical signal flowing through the conductive substrate 11 is transmitted to the outside by the flexible printed substrate 16 via the conductive foil 162, it is possible to transmit the electrical signal from the living body 14 to the outside.

In the example shown in Fig. 3A, the covering wire 15 having a predetermined thickness, which is a signal-transmitting member 13, is disposed on the upper surface side of the conductive substrate 11. Therefore, when the biological electrode 1 is mounted to the living body 14, there is a case where an uneven load based on the thickness of the covering wire 15 is applied to the conductive rubber body 12 that is in direct contact with the living body 14 (see Fig. 2, hereinafter, the same) and the living body 14 feels unevenness on the mounting portion. In contrast, in the example shown in Fig. 3B, the surface of the thin plate-shaped flexible printed substrate 16, which is a signal-transmitting member 13, is substantially flush with the surface of the conductive substrate 11. Thus, since the load based on the thickness of the flexible printed substrate 16 is less likely to be applied to the conductive rubber body 12, even if the biological electrode 1 is mounted for a long period of time, the living body hardly feels the unevenness in the mounting portion, the discomfort is reduced, and the biological electrode 1 can realize weight reduction and miniaturization.

Fig. 4 is a diagram showing another configuration example of the biological electrode obtained by the method for manufacturing the biological electrode according to an embodiment of the present invention. In the example shown in Fig. 4, the biological electrode 2 comprises an insulating layer 17 provided on the conductive substrate 11. The insulating layer 17 contains insulating rubber. In the biological electrode 2, even if the insulating layer 17 is provided on the conductive substrate 11, the surface of the conductive substrate 11 is not in direct contact with the living body 14, so that the transmission of electric signals from the living body 14 is not hindered by the insulating layer 17. Then, when the insulating layer 17 is provided, as shown in Fig. 3B, by providing the flexible printed substrate 16 as the signal transmitting member 13, the surface of the flexible printed substrate 16 is substantially flush with the surface of the conductive substrate 11, so that it is possible to stably hold the insulating layer 17 and prevent the curvature of the biological electrode 2. Hereinafter, various components of the biological electrode will be described in detail.

The conductive rubber body 12 contains an additive such as silicone rubber, silver powder, and, if necessary, a dispersant. Examples of the silicone rubber contained in the conductive rubber body 12 include a room temperature-curable liquid silicone rubber. The room temperature-curable liquid silicone rubber is a silicone rubber which is in a liquid state or a paste state before curing, and usually undergoes a curing reaction at 20°C to 100°C to become a rubber elastic body. The curing reaction may proceed gradually by moisture in air or immediately by adding a curing agent to the main agent, and any type of the curing reaction may be used in the present invention. In some cases, the reaction of curing by moisture is classified as 1-component type, and the reaction of curing by adding the curing agent is classified as 2-components type.

As the room temperature-curable liquid silicone rubber, a commercially available product can be used. For example, as the liquid silicone rubber, a trade name "KE-106" (manufactured by Shin-Etsu Chemical Co., Ltd.), a trade name "CAT-RG" (manufactured by Shin-Etsu Chemical Co., Ltd.), or the like can be used. In addition, as the liquid silicone rubber, a kind of silicone rubber may be used alone, or a plurality of kinds may be mixed and used.

The conductive rubber body 12 preferably contains 50 to 600 parts by mass, preferably 100 to 400 parts by mass, of silver powder with respect to 100 parts by mass of silicone rubber from the viewpoint of improving conductivity.

The silver powder contained in the conductive rubber body 12 may contain at least one selected from aggregated silver powder, flaked silver powder, and the like. As the silver powder contained in the conductive rubber body 12, a mixed silver powder composed of silver powder and fumed silica, which is obtained by adding fumed silica to an aggregated silver powder and mixing, is at least used. In the mixed silver powder, the aggregated silver powder and the fumed silica are mixed and dispersed by a disperser, and a part of the aggregated silver powder may be crushed and finely divided. For example, a part of the aggregated silver powder used for the preparation of the mixed silver powder may become primary particles or finer particles in the vicinity of the primary particles by crushing and dispersing, and may be dispersed in the conductive rubber body 12 in the state of such finer particles. Further, the fumed silica is mixed with the silver powder to effectively suppress reaggregation of the silver powder, and to improve the fluidity of the silver powder.

The aggregated silver powder is one in which a plurality of particulate primary particles are aggregated in a three-dimensional shape. Examples of the aggregated silver powder include a trade name "G-35" (manufactured by DOWA Electronics Materials Co., Ltd.).

The flaked silver powder refers to one having a scaly shape, and examples thereof include a trade name "FA-D-3" and a trade name "FA-2-3" (both manufactured by DOWA Electronics Materials Co., Ltd.).

The average particle diameter of the silver powder is not particularly limited as long as it can impart conductivity to the conductive rubber body 12. For example, the average particle diameter of the aggregated silver powder is preferably 4µm or more and 8µm or less. The average particle diameter of the flaked silver powder is preferably 5µm or more and 15µm or less. The average particle diameter described above is the average particle diameter of the silver powder in the primary particles. The average particle diameter of the silver powder is an average diameter measured by an electron micrograph and calculated by an arithmetic average.

The blending ratio of the aggregated silver powder and the flake silver powder is not particularly limited. For example, the blending ratio (mass ratio) of the aggregated silver powder and the flaked silver powder is preferably 1:5 to 5:1

Fumed silica is composed of minute nanometer-sized silica particles. The silver powder contained in the conductive rubber body 12 is added to the liquid silicone rubber in a state of a mixed silver powder obtained by adding fumed silica to an aggregated silver powder and mixing (that is, a mixed powder composed of silver powder and fumed silica). In the mixed silver powder, silver powder particles and fumed silica are mixed to suppress aggregation of silver powder particles. Therefore, the mixed silver powder composed of silver powder and fumed silica has extremely excellent fluidity in a liquid silicone rubber. The excellent fluidity of the mixed silver powder facilitates the handling of the material for producing the conductive rubber body 12. In addition, the dispersibility inside the rubber when blended into the silicone rubber is improved, the familiarity with the rubber is improved, and the strain resistance of the conductive rubber body 12 is improved. As a result, the resistance value of the biological electrode 1 is prevented from rising over time due to strain.

Generally, fumed silica is roughly classified into hydrophobic fumed silica and hydrophilic fumed silica. As the fumed silica, hydrophobic fumed silica is preferably used. Examples of the hydrophobic fumed silica include a trade name "AEROSIL R972" (manufactured by Nippon Aerosil Co., Ltd.) and the like. Examples of the hydrophilic fumed silica include a trade name "AEROSIL 200" (manufactured by Nippon Aerosil Co., Ltd.) and the like.

The blending amount of the silver powder can be appropriately set within a range capable of imparting conductivity. For example, the silver powder is preferably in the range of 50 to 500 parts by mass, and particularly preferably in the range of 100 to 300 parts by mass, per 100 parts by mass of the liquid silicone rubber. Note that at least a part of the silver powder is blended into the liquid silicone rubber in a state of a mixed silver powder containing fumed silica. Therefore, the blending amount of the mixed silver powder containing the silver powder is determined in consideration of the addition amount of the fumed silica described above.

In addition to the above-described components, the conductive rubber body 12 may further include other components as long as the effects of the present invention are not impaired. As other components, for example, a reinforcing agent, a filler such as dry silica, a compounding agent commonly used in the rubber industry such as an antioxidant, a processing aid, and a plasticizer can be appropriately blended.

The conductive rubber body 12 may further contain a modified silicone as a dispersant. As the modified silicone, one in which a side chain resulting in modification is introduced into a main chain composed of a siloxane bond (-Si-O-; also referred to as a silicone chain) can be preferably used, and examples thereof include silicones including polyether modification, polyether-alkyl co-modification, polyglycerin modification, and polyglycerin-alkyl co-modification. The side chain resulting in modification preferably includes an ether bond (-C-O-C-).

As the polyether-modified silicone, one in which a side chain composed of a polyether chain is introduced into a main chain composed of a silicone chain can be used. As the polyether-alkyl co-modified silicone, one in which a side chain composed of a polyether chain and a side chain composed of an alkyl chain are introduced into a main chain composed of a silicone chain can be used. As the polyglycerin-modified silicone, one in which a side chain composed of a polyglycerin chain is introduced into a main chain composed of a silicone chain can be used. As the polyglycerin-alkyl co-modified silicone, one in which a side chain composed of a polyglycerin chain and a side chain composed of an alkyl chain are introduced into a main chain composed of a silicone chain can be used. Among the above, the polyether-modified silicone or the polyglycerin-modified silicone is particularly preferable.

The thickness of the conductive rubber body 12 is not particularly limited as long as it can impart conductivity to the conductive rubber body 12. The thickness of the conductive rubber body 12 is preferably 18µm or more and 80µm or less from the viewpoint of improving the conductivity of the biological electrode and ensuring the flexibility of the biological electrode, and more preferably 30µm or more and 60µm or less from the viewpoint of improving the adhesion between the conductive substrate 11 and the conductive rubber body 12 to prevent peeling of the conductive rubber body 12 and reducing the contact impedance with the living body.

The conductive substrate 11 is an optional component for supporting the conductive rubber body 12. The material of the conductive substrate 11 is not particularly limited as long as it has conductivity. For example, the conductive silicone rubber configured in the same manner as the conductive rubber body 12 can be used. The details of the conductive silicone rubber configured in the same manner as the conductive rubber body 12 have been described above. As the material of the conductive substrate 11, the conductive silicone rubber containing silicone rubber and conductive particles can also be used. The conductive substrate 11 contains the conductive silicone rubber, whereby the adhesion between the conductive substrate 11 and the conductive rubber body 12 can be improved. As the silicone rubber, a liquid silicone rubber is preferable, and for example, an organosilicon polymer is used. As the organosilicon polymer, one having a siloxane bond (-Si-O-) as a main chain and having a hydrocarbon group such as a methyl group, a phenyl group, or a vinyl group or a hydrogen as a side chain is preferable. As the silicone rubber, a silicone rubber of an addition reaction type may be used, and a silicone rubber of a condensation reaction type may be used. The silicone rubber of the addition reaction type is a silicone rubber which is cured by an addition reaction, and examples thereof include a silicone rubber having a hydrogen or a vinyl group as a side chain. In addition, the silicone rubber of the condensation reaction type is a silicone rubber which is cured by a condensation reaction, and examples thereof include a silicone rubber having a hydroxyl group at its terminal. Among the above, the silicone rubber of an addition reaction type is preferable from the viewpoint of more suitably maintaining adhesion to the conductive rubber body 12. One type of these silicone rubbers may be used alone, or two or more types thereof may be used in combination.

As the conductive particles, conductive carbon particles such as various carbon blacks and the like are used. The conductive carbon particles are not limited as long as they can impart conductivity to the conductive substrate 11. Examples of the conductive carbon particles include various carbon particles such as carbon black and graphite. Examples of the carbon black include Ketjen black and acetylene black. One kind of these may be used alone, or two or more kinds thereof may be used in combination. Among the above, Ketjen black is preferable as the carbon black from the viewpoint of improving conductivity of the conductive substrate 11.

The average particle diameter of the conductive particles is not particularly limited as long as it can impart conductivity to the conductive substrate 11. The average particle diameter of the conductive particles is preferably 0.1µm or more and 100µm or less, and more preferably 1µm or more and 30µm or less, from the viewpoint of improving the conductivity of the conductive substrate 11 and ensuring the flexibility of the conductive substrate 11. The average particle diameter of the conductive particles is an average diameter measured by an electron micrograph and calculated by an arithmetic average.

The content of the conductive particles in the conductive substrate 11 is not particularly limited as long as it can impart conductivity to the conductive substrate 11. The content of the conductive particles in the conductive substrate 11 is preferably 10% by mass or more and 70% by mass or less, more preferably 20% by mass or more and 50% by mass or less, with respect to the total mass of the conductive substrate 11, from the viewpoint of improving the conductivity of the conductive substrate 11 and ensuring the flexibility of the conductive substrate 11.

As the conductive silicone rubber contained in the conductive substrate 11, for example, a commercially available product such as a trade name "KE-3801M-U" (manufactured by Shin-Etsu Chemical Co., Ltd.) may be used.

In addition, the conductive substrate 11 may be obtained by crosslinking the above-described conductive silicone rubber by a crosslinking agent. As the crosslinking agent, for example, a commercially available product such as a trade name "C-8A" (2,5-dimethyl-2,5-bis(t-butylperoxy)hexane content of 80% by mass, manufactured by Shin-Etsu Chemical Co., Ltd.) may be used.

The thickness of the conductive substrate 11 is not particularly limited as long as it can ensure the flexibility of the conductive substrate 11, and is preferably 0.1mm or more and 2mm or less, more preferably 0.4mm or more and 1.5mm or less.

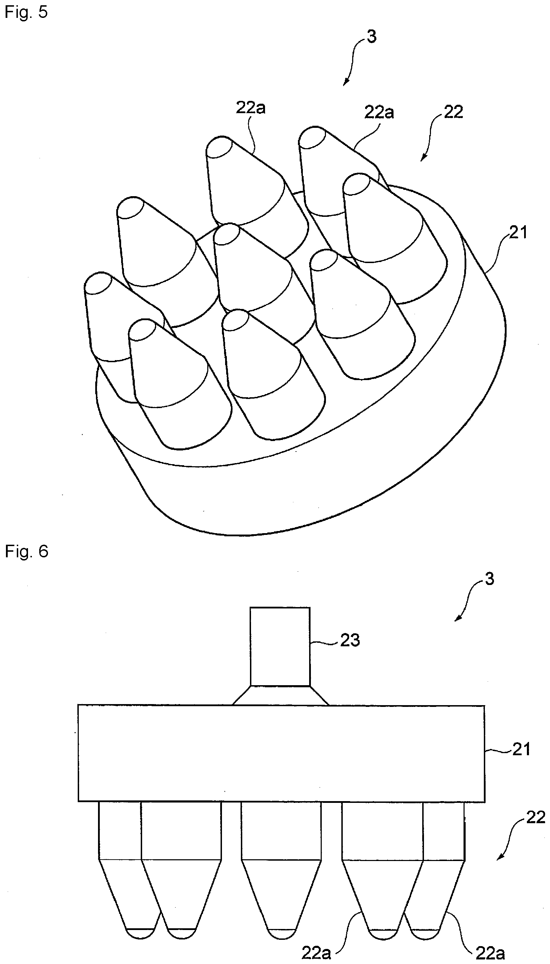

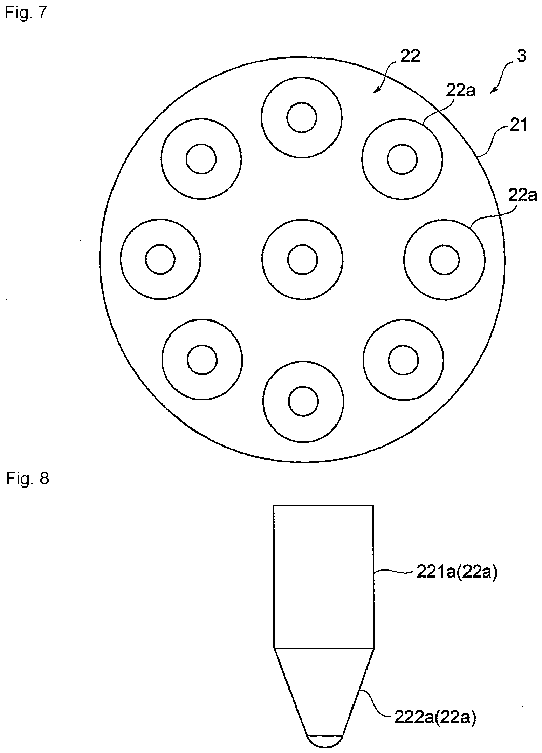

Figs. 5 to 8 are views showing another example of the biological electrode obtained by the method for manufacturing a biological electrode according to the present embodiment; Fig. 5 is a perspective view of the biological electrode 3, Fig. 6 is a side view of the biological electrode 3, Fig. 7 is a top view of the biological electrode 3, and Fig. 8 is a partially side view showing only the protruding body 22a constituting the biological electrode 3.

As shown in Figs. 5 to 7, the biological electrode 3 is made of a conductive rubber body having a disk 21 and a protrusion 22 provided on one surface of the disk 21. As such a conductive rubber body, the conductive silicone rubber configured in the same manner as the conductive rubber body 12 shown in Fig. 1 can be used. The protrusion 22 has a brushed shape composed of a plurality of protruding bodies 22a. The protrusion 22 is composed of a protruding body 22a disposed at the center of the disk 21, and a plurality of protruding bodies 22a disposed on the circumference around the protruding body 22a as the center. On the other main surface of the disk 21, the connecting member 23 is provided.

As shown in Fig. 8, the protruding body 22a includes a main body 221a and a tip portion 222a provided on the main body 221a. The main body 221a has a substantially cylindrical shape, and one end side thereof is provided on the disk 21. The tip portion 222a has a generally conical shape having a hemispherical shape on the apex side, and the bottom surface is provided on the surface of the other end side of the main body 221a.

In the biological electrode 3 shown in Figs. 5 to 8, a plurality of protruding bodies 22a serve as terminals having a brushed shape and come into contact with the human body. At this time, since the tip portion 222a of the protruding body 22a has a hemispherical shape on the apex side, no discomfort occurs when it comes into contact with the skin, and the adhesion to the skin is also improved due to the flexibility of the protruding body 22a. In addition, since the surface in contact with the skin has conductivity due to silver, the contact impedance is low, and stable measurement can be performed even in measurement under dry conditions in which paste or the like is not used. The present embodiment is also applicable to the biological electrode 3 provided with the conductive rubbers having such terminals having a brushed shape.

### <Method for manufacturing biological electrode>

The method for manufacturing a biological electrode according to the embodiments of the present invention is a manufacturing method of the biological electrode includes a step of adding fumed silica to an aggregated silver powder and mixing to obtain a mixed silver powder in which the silver powder and the fumed silica are dispersed, and a step of forming a conductive rubber body containing silicone rubber and the mixed silver powder.

In the step of obtaining a mixed silver powder containing silver powder and fumed silica, the aggregated silver powder and the fumed silica are mixed and dispersed by a disperser, for example, and a part of the aggregated silver powder may be crushed and finely divided. For example, a part of the aggregated silver powder used for the preparation of the mixed silver powder may become primary particles or finer particles in the vicinity of the primary particles by crushing and dispersing, and may be contained in the mixed silver powder in the state of such finer particles. Further, the fumed silica is mixed with the silver powder to effectively suppress reaggregation of the silver powder, and to improve the fluidity of the silver powder (in other words, the mixed silver powder). By containing such mixed silver powder in the silicone rubber to form a conductive rubber body, the silver powder is uniformly dispersed in the conductive rubber body constituting the biological electrode, and an increase in resistance value over time due to strain can be effectively suppressed. In addition, the conductive rubber body constituting the biological electrode has good adhesion to the body of a subject for detection, does not easily cause discomfort even if the conductive rubber body is in close contact with the body for a long time with a soft skin touch, and can maintain stable contact with the body of the subject for detection. Further, even in a three-dimensional shape such as a brush, it expresses high conductivity, and it is possible to stably measure with little noise. In addition, since it is not necessary to use a gel or the like, it can be stably measured even in measurement under dry conditions, and the method of use is also simple. Note that, as for the conductivity of the obtained conductive rubber body, there is no effect due to the presence or absence of the addition of fumed silica, and the biological electrode having excellent conductivity can be produced while exhibiting the above-described effects.

The step of obtaining the mixed silver powder is a step of preparing an aggregated silver powder and fumed silica, adding the fumed silica to the aggregated silver powder, and dispersing them in advance by a disperser or the like. As described above, the mixed silver powder is preferably mixed and dispersed by a disperser. Incidentally, the aggregated silver powder and fumed silica may be first mixed by hand or by a kneader or the like, and then the aggregated silver powder may be crushed and dispersed by a disperser. By this step, the aggregated silver powder becomes primary particles or finer particles in the vicinity of the primary particles. There is no particular limitation on the disperser, and for example, a known instrument for pulverizing a powder such as a household mill can be used. The dispersion time by the disperser is not particularly limited, and it is preferable that the dispersion be performed in such a time to form a stable dispersion system. For example, the dispersion time may be from 1 to 10 minutes.

Examples of the aggregated silver powder include a trade name "G-35" (manufactured by DOWA Electronics Materials Co., Ltd.). The average particle diameter of the silver powder is not particularly limited as long as it can impart conductivity to the conductive rubber body 12. The average particle diameter of the silver powder is preferably 4µm or more and 8µm or less.

As the fumed silica, hydrophobic fumed silica may be used, or hydrophilic fumed silica may be used, but hydrophobic fumed silica is more preferably used. When hydrophobic fumed silica is used, it is considered that the dispersibility of the silver powder is further increased and the handling property is further improved as compared with hydrophilic fumed silica. Examples of the hydrophobic fumed silica include a trade name "AEROSIL R972" (manufactured by Nippon Aerosil Co., Ltd.) and the like. Examples of the hydrophilic fumed silica include a trade name "AEROSIL 200" (manufactured by Nippon Aerosil Co., Ltd.) and the like.

Although there is no particular limitation on the blending ratio of the aggregated silver powder and the fumed silica, for example, it is preferable that the fumed silica is 1 to 5 parts by mass per 100 parts by mass of the aggregated silver powder.

The step of forming the conductive rubber body containing the silicone rubber and the mixed silver powder is a step of mixing the silicone rubber and the mixed silver powder obtained by the above step to form a conductive rubber body having a desired shape. In the step of forming the conductive rubber body, in addition to the mixed silver powder described above, it is preferable to further add flaked silver powder to form the conductive rubber body. Examples of the flaked silver powder include a trade name "FA-D-3" and a trade name "FA-2-3" (both manufactured by DOWA Electronics Materials Co., Ltd.). The blending ratio (mass ratio) of the aggregated silver powder and the flaked silver powder is preferably 1:5 to 5:1.

In the step of forming the conductive rubber body, (a) the conductive rubber body may be formed on the conductive substrate, or (b) the conductive rubber body may be formed alone. When (a) the conductive rubber body is formed on the conductive substrate, for example, examples of the method include a method in which a liquid silicone rubber, a mixed silver powder, and, if necessary, a silver powder-containing paste containing a crosslinking agent, a modified silicone, or the like is applied on a conductive substrate, and then the silver powder-containing paste is heated and cured. A material in which the silver powder-containing paste is cured becomes a conductive rubber body. In addition, when (b) the conductive rubber body is formed alone, examples of the method include a method in which the above-described silver powder-containing paste is injected into a forming mold having a predetermined internal shape, and then the silver powder-containing paste is heated and cured in the forming mold. Typically, in the case of (a) above, a layered conductive rubber body can be formed on the conductive substrate, and in the case of (b) above, a conductive rubber body having a desired shape such as a brushed shape can be formed.

Hereinafter, examples of the step of forming the conductive rubber body will be described in detail. When (a) the conductive rubber body is formed on the conductive substrate, a conductive substrate is first prepared. The conductive substrate may be one using a commercially available material or one newly produced. When a conductive substrate is newly produced, for example, a conductive silicone rubber containing a predetermined amount of conductive particles and a crosslinking agent are kneaded by a kneading machine such as a kneader and a roll at room temperature (20°C or more and 40°C or less) for 1 minute or more and 1 hour or less to obtain a substrate. Thereafter, the kneaded substrate is subjected to primary crosslinking under conditions of 100°C or more and 300°C or less and 1 minute or more and 1 hour or less, and then subjected to secondary crosslinking under conditions of 150°C or more and 350°C or less and 1 hour or more and 10 hours or less to produce a conductive substrate.

Next, a silver powder-containing paste is prepared by stirring a liquid silicone rubber, a mixed silver powder, a crosslinking agent, or the like for a predetermined time by a mixer or the like. The temperature at the time of stirring may be, for example, room temperature (20°C or more and 40°C or less). The stirring time may be, for example, 1 minute or more and 1 hour or less. Next, the silver powder-containing paste is applied onto the conductive substrate. For example, immersion, spraying, roll coater, flow coater, ink jet, screen printing, or the like are used to apply the silver powder-containing paste onto the conductive substrate. The coating thickness of the silver powder-containing paste is preferably 25µm or more and 200µm or less, and more preferably 35µm or more and 100µm or less. Thus, since the adhesion of the conductive rubber body to the conductive substrate can be increased, peeling of the conductive rubber body from the conductive substrate can be easily prevented, and the contact impedance can be lowered. Then, curing of the silver powder-containing paste is performed. In this step, for example, curing of the silver powder-containing paste can be performed by heating the silver powder-containing paste to a predetermined temperature for a predetermined time. The heating time of the silver powder-containing paste is preferably 10 to 120 minutes, more preferably 15 to 90 minutes, and still more preferably 20 to 45 minutes. Further, the heating temperature of the silver powder-containing paste is preferably 100 to 200°C, more preferably 120 to 180°C, and still more preferably 140 to 160°C. The silver powder-containing paste can be cured by heating, for example, at 150°C for 30 minutes.

When (b) the conductive rubber body is formed alone, a silver powder-containing paste is prepared in the same manner as in the case of (a) above. Next, after injecting the silver powder-containing paste into a mold corresponding to a desired shape such as a brushed shape, primary crosslinking by press crosslinking is performed. This is followed by further secondary cross-linking. Specifically, the kneaded substrate is primary crosslinked under the conditions of 100°C or more and 300°C or less and 1 minute or more and 1 hour or less, and then secondary crosslinked under the conditions of 150°C or more and 350°C or less and 1 hour or more and 10 hours or less to produce a conductive rubber body.

After forming the conductive rubber body as described above (a) or (b), the conductive rubber body may be immersed in an inorganic salt-containing solution having a temperature of 70°C or more and 180°C or less. During immersion in the inorganic salt-containing solution, an inorganic salt, an anion derived from the inorganic salt, and a cation effectively penetrate into the conductive rubber body. It is preferable that the inorganic salt-containing solution contains an inorganic salt, a solvent for dissolving the inorganic salt, and, if necessary, other additives.

The inorganic salt contained in the inorganic salt-containing solution is not particularly limited as long as it can penetrate into the conductive rubber body, but at least one inorganic salt selected from the group consisting of chloride salt, sulfide salt, and carbonate is preferably used. As the inorganic salt, it is more preferable to use at least one inorganic salt selected from the group consisting of sodium chloride, potassium chloride, lithium chloride, calcium chloride, magnesium chloride, sodium sulfate, potassium sulfate, lithium sulfate, calcium sulfate, magnesium sulfate, sodium carbonate, potassium carbonate, lithium carbonate, calcium carbonate, and magnesium carbonate. One kind of these inorganic salts may be used alone, and two or more kinds thereof may be used in combination. Among the above, as the inorganic salt, a chloride salt is preferable from the viewpoint of solubility in a solvent and ion mobility, and a chloride salt by an alkali metal such as sodium chloride, potassium chloride and lithium chloride is more preferable, and sodium chloride is further preferable from the viewpoint of low cost, safety to the human body, and ion exchange property with salinity contained in sweat of the human body. Further, the conductive rubber body can contain a halide ion, a sulfate ion, and a carbonate ion as an anion derived from an inorganic salt. As the halide ion, chloride ion (Cl⁻) is preferable. The conductive rubbers may contain Li⁺, Na⁺, K⁺, Mg²⁺, Ca²⁺ or the like as a cation derived from an inorganic salt. Such an inorganic salt or an anion or a cation derived from an inorganic salt can penetrate into the conductive rubber body to suppress potential fluctuation based on the polarization voltage in the conductive rubber body, thereby effectively reducing the potential fluctuation noise. The concentration of the inorganic salt in the inorganic salt-containing solution is preferably 0.1mol/L or more, more preferably 0.5mol/L or more and 20mol/L or less. When the concentration of the inorganic salt in the inorganic salt-containing solution is within these ranges, it is possible to effectively penetrate the inorganic salt and the anion or the cation derived from the inorganic salt into the conductive rubber body.

There is no particular limitation on the solvent contained in the inorganic salt-containing solution as long as it can dissolve the inorganic salt. Examples of the solvent include ketones such as water and acetone, and alcohols such as methanol and ethanol. One kind of these solvents may be used alone, or two or more kinds thereof may be used in combination. Among the above, water, ethanol, or a mixture of water and ethanol is preferable from the viewpoint of safety and low-cost, and water is preferable from the viewpoint of safety and low-cost.

In the step of immersing the conductive rubber body in an inorganic salt-containing solution, it is preferable that the conductive rubber body is immersed in the inorganic salt-containing solution under a pressure condition. More preferably, the conductive rubber body is immersed in the inorganic salt-containing solution under a pressure of 1 atm or more and 10 atm or less, more preferably under a pressure of 2 atm or more and 3 atm or less. By immersing the conductive rubber body in the inorganic salt-containing solution under pressure conditions, it is possible to further increase the rate of penetration of an inorganic salt, an anion or a cation derived from an inorganic salt, into the conductive rubber body and to effectively increase the concentration of an inorganic salt, an anion, or a cation in the conductive rubber body. As a result, the potential fluctuation noises of the biological electrode can be effectively reduced. There is no particular limitation on the method of pressurizing, and examples thereof include a method in which a conductive rubber body is placed in a sealed container (e.g., an autoclave) in which an inorganic salt-containing solution is injected, and then a pressure in the sealed container is increased.

The time for immersing the conductive rubber body in the inorganic salt-containing solution can be appropriately changed depending on the temperature of the inorganic salt-containing solution, the concentration of the inorganic salt in the inorganic salt-containing solution, and the pressure at the time of pressurizing. The immersion time of the conductive rubber body in the inorganic salt-containing solution is preferably 15 minutes or more and 180 minutes or less, more preferably 30 minutes or more and 120 minutes or less, and still more preferably 45 minutes or more and 90 minutes or less, from the viewpoint of efficiently dispersing an inorganic salt, an anion or a cation derived from an inorganic salt in the conductive rubber body.

As described above, the method for manufacturing a biological electrode of the present embodiment includes a step of adding fumed silica to an aggregated silver powder and dispersing it in advance by a disperser or the like, and the mixed silver powder obtained by the step is added to the silicone rubber to form a conductive rubber body.

### (Rubber for biological electrode, and Rubber Electrode)

Next, a rubber for the biological electrode according to the embodiment of the present invention will be described. The rubber for the biological electrode according to the present embodiment is a rubber for the biological electrode containing at least silicone rubber, aggregated silver powder, and fumed silica. In addition, the rubber for the biological electrode contains 1 to 5 parts by mass of fumed silica per 100 parts by mass of the aggregated silver powder. The aggregated silver powder does not contain a flaked silver powder having a scaly shape, and examples thereof include silver powder in which a plurality of particulate primary particles are aggregated in a three-dimensional shape. Preferably, the rubber for the biological electrode further contains a flaked silver powder. The blending ratio (mass ratio) of the aggregated silver powder and the flaked silver powder is preferably 1:5 to 5:1. Examples of the rubber for the biological electrode according to the present embodiment include a conductive rubber body manufactured by the manufacturing method of the biological electrode described above. In the rubber for the biological electrode according to the present embodiment, an increase in resistance value over time due to strain is suppressed. Next, a rubber electrode according to the embodiment of the present invention will be described. The rubber electrode according to the present embodiment is a rubber electrode made of the rubber for the biological electrode described above. Examples of the rubber electrode according to the embodiment include the biological electrode manufactured by the manufacturing method of the biological electrode described above.

### (Examples)

Hereinafter, the present invention will be described in more detail based on examples performed in order to clarify the effect of the present invention. Note that the present invention is not limited in any way by the following examples and comparative examples.

### <Preparation of Mixed silver powder>

A sample mixed in the following blending amount was dispersed with a disperser (household mill) for 2 minutes to prepare mixed silver powders 1 to 4 containing silver powder and fumed silica. Note that the pure silver particles described below ("G-35" manufactured by DOWA Electronics Materials Co., Ltd.) used for preparing the mixed silver powders 1 to 4 were aggregated silver powders at the time of preparation thereof.

### (Blended components of Mixed silver powder 1)

·Pure silver particles ("G-35", manufactured by DOWA Electronics Materials Co., Ltd.), 100 parts by mass
·Hydrophobic fumed silica ("G-35AEROSIL R972", manufactured by Nippon Aerosil Co., Ltd., average primary particle diameter: 16nm), 1 part by weight

### (Blended components of Mixed silver powder 2)

·Pure silver particles ("G-35", manufactured by DOWA Electronics Materials Co., Ltd.), 100 parts by mass
·Hydrophilic fumed silica ("G-35AEROSIL 200", manufactured by Nippon Aerosil Co., Ltd., average primary particle diameter: 12nm), 1 part by weight

### (Blended components of Mixed silver powder 3)

·Pure silver particles ("G-35", manufactured by DOWA Electronics Materials Co., Ltd.), 100 parts by mass
·Hydrophobic fumed silica ("G-35AEROSIL R972", manufactured by Nippon Aerosil Co., Ltd., average primary particle diameter: 16nm), 2 parts by mass

### (Blended components of Mixed silver powder 4)

·Pure silver particles ("G-35", manufactured by DOWA Electronics Materials Co., Ltd.), 100 parts by mass
·Hydrophilic fumed silica ("G-35AEROSIL 200", manufactured by Nippon Aerosil Co., Ltd., average primary particle diameter: 12nm), 2 parts by mass

### <Sieve passage rate test of Mixed silver powder>

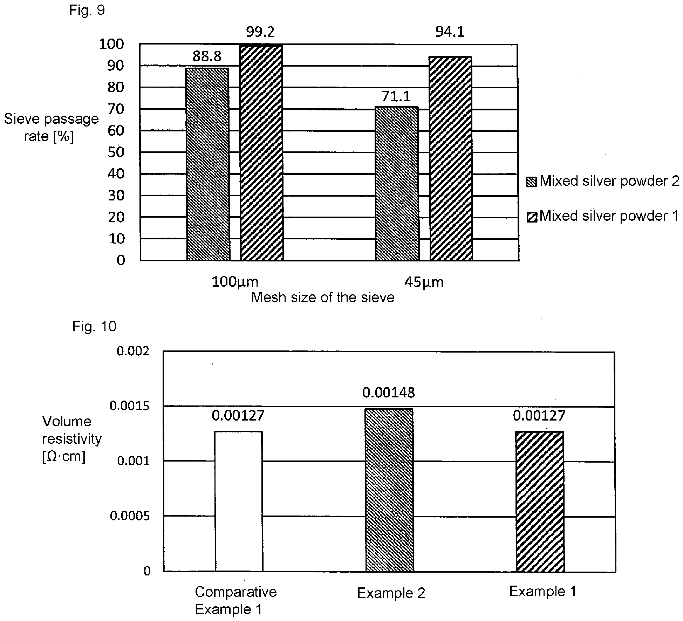

Each of the mixed silver powders 1 and 2 was sieved using a sieve with a mesh size of 45µm or 100µm to calculate a mass ratio of the powder passing through each sieve (hereinafter, referred to as "sieve passage rate"). Incidentally, sieving the respective powder was carried out by beating the side surface of the sieve with a wooden hammer while shaking the sieve by hand. The results of the sieve passage rate test are shown in Fig. 9.

Fig. 9 is a graph showing the results of the sieve passage rate test of the mixed silver powders 1 and 2. In Fig. 9, the vertical axis represents the sieve passing rate [%], and the horizontal axis represents the mesh size of the sieve. Fig. 9 shows a test result using a sieve having a mesh size of 45µm on the right side of the graph, and shows a test result using a sieve having a mesh size of 100µm on the left side of the graph.

As shown in Fig. 9, each of mixed silver powders 1 and 2 in which fumed silica was blended with pure silver particles exhibited a good sieve passage rate in sieving using a sieve with a mesh size of 45µm or 100µm. In particular, the mixed silver powder 1 blended with hydrophobic fumed silica had extremely high sieve passage rate in each sieving, and had very good handling property.

### (Example 1)

The following blended components were mixed for 30 seconds with a centrifugal stirrer, press-crosslinked (primary crosslinked) at 150°C for 10 minutes, and then oven-crosslinked (secondary crosslinked) at 150°C for 30 minutes to produce a biological electrode of Example 1 consisting of silicone rubber. The biological electrode of Example 1 was formed to have a cylindrical shape having a diameter of 10mm at the end face and a length of 5mm in the axial direction. Further, the prroduced biological electrode was immersed in a 10%NaCl aqueous solution, and heated and pressurized at 121°C and 2 atm for 1 hour. The biological electrode of Example 1 is referred to as "conductive rubber body 1". Pure silver particles ("FA-2-3" manufactured by DOWA Electronics Materials Co., Ltd.) in the blended component described below were flaked silver powder at the time of preparation thereof.

### (Blended components of Conductive rubber body 1)

·Room temperature-curable liquid silicone rubber ("KE-106" and "CAT-RG", manufactured by Shin-Etsu Chemical Co., Ltd., were mixed at 10:1.), 100 parts by mass
·Mixed silver powder 3 described above, 150 parts by mass
·Pure silver particles ("FA-2-3", manufactured by DOWA Electronics Materials Co., Ltd.), 150 parts by mass
·Silicone oil 1 ("KF-6015", manufactured by Shin-Etsu Chemical Co., Ltd.), 10 parts by mass
·Silicone oil 2 ("KF-6106", manufactured by Shin-Etsu Chemical Co., Ltd.), 10 parts by mass

### (Example 2)

A biological electrode of Example 2 was produced in the same manner as in Example 1, except that the following blended components were changed to the following blended components. The biological electrode of Example 2 is referred to as "conductive rubber body 2 ".

### (Blended components of Conductive rubber body 2)

·Room temperature-curable liquid silicone rubber ("KE-106" and "CAT-RG", manufactured by Shin-Etsu Chemical Co., Ltd., were mixed at 10:1.), 100 parts by mass
·Mixed silver powder 4 describe above, 150 parts by mass
·Pure silver particles ("FA-2-3", manufactured by DOWA Electronics Materials Co., Ltd.), 150 parts by mass
·Silicone oil 1 ("KF-6015", manufactured by Shin-Etsu Chemical Co., Ltd.), 10 parts by mass
·Silicone oil 2 ("KF-6106", manufactured by Shin-Etsu Chemical Co., Ltd.), 10 parts by mass

### (Comparative Example 1)

A biological electrode of Comparative Example 1 was produced in the same manner as in Example 1, except that the following blended components were changed to the following blended components. The biological electrode of Comparative Example 1 is referred to as "conductive rubber body 3".

### (Blended components of Conductive rubber body 3)

·Room temperature-curable liquid silicone rubber ("KE-106" and "CAT-RG", manufactured by Shin-Etsu Chemical Co., Ltd., were mixed at 10:1.), 100 parts by mass
·Pure silver particles ("G-35", manufactured by DOWA Electronics Materials Co., Ltd.), 150 parts by mass
·Pure silver particles ("FA-2-3", manufactured by DOWA Electronics Materials Co., Ltd.), 150 parts by mass
·Silicone oil 1 ("KF-6015", manufactured by Shin-Etsu Chemical Co., Ltd.), 10 parts by mass
·Silicone oil 2 ("KF-6106", manufactured by Shin-Etsu Chemical Co., Ltd.), 10 parts by mass

### <Volume resistivity>

The volume resistivity [Ω·cm] of the biological electrode in Examples 1 and 2 and Comparative Example 1 was measured by the following measuring method. The measurement result of the volume resistivity is shown in Fig. 10. Note that the value of the volume resistivity shown in Fig. 10 is a value obtained by quickly measuring without applying a load or the like to the respective biological electrode after producing the respective biological electrode.

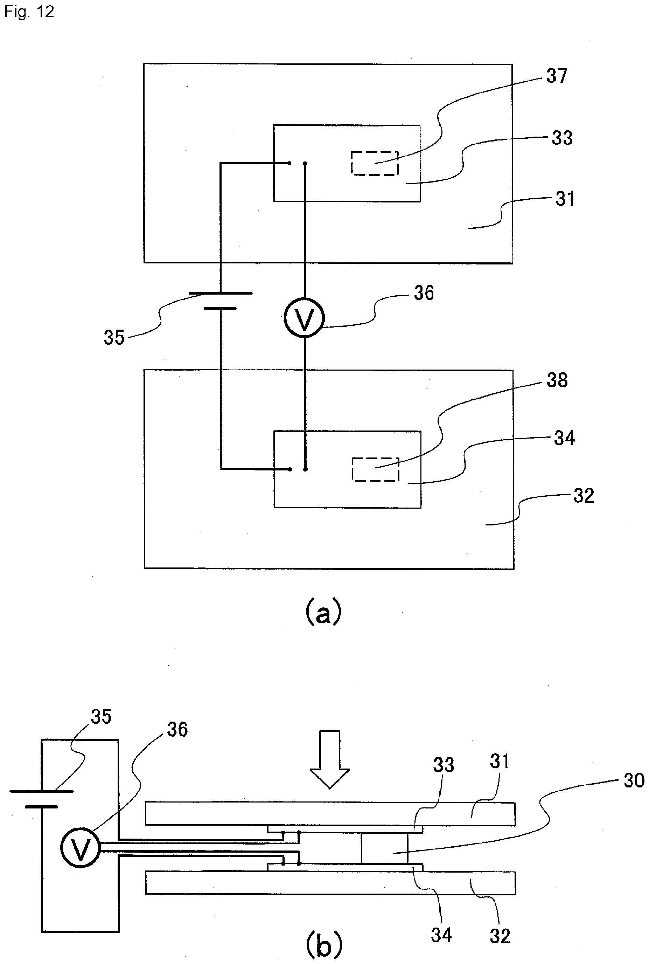

The volume resistivity of the biological electrode was measured using plate-shaped resistivity measuring members 31 and 32 as shown in Fig. 12. Fig. 12 is an explanatory drawing for explaining a method of measuring the volume resistivity of the biological electrode; Fig. 12(a) shows a plan view of a resistivity measuring member for measuring the volume resistivity of a biological electrode. Fig. 12(b) is a side view for explaining a method of measuring the volume resistivity of a biological electrode. The resistivity measuring members 31 and 32 are a plate-shaped member as a set by two sheets, and measuring electrodes 33 and 34 formed by gold plating are disposed on each surface. The measuring electrodes 33 and 34 were 20mm in length and 40mm in width. In Fig. 12(a), the ranges surrounded by dashed lines indicated by reference numerals 37 and 38 are sample placement positions for placing the biological electrode 30 (see Fig. 12(b)), which is a sample. As shown in Fig. 12(b), when measuring the volume resistivity of the biological electrode 30, the biological electrode 30 was sandwiched between the two plate-shaped resistivity measuring members 31 and 32, a constant current of 100mA was applied to the two resistivity measuring members 31 and 32, and the potential difference between the measuring electrodes 33 and 34 placed facing each other with the biological electrode 30 therebetween was measured. In Fig. 12, reference numeral 35 denotes a constant current power supply for applying a constant current, and reference numeral 36 denotes a voltmeter for measuring the potential difference. The volume resistivity of the biological electrode 30 was calculated based on Ohm's law from the current value applied by the constant current power supply 35, the potential difference measured by the voltmeter 36, and the cross-sectional area and thickness of the biological electrode 30. When measuring the potential difference between the measuring electrodes 33 and 34 as shown in Fig. 12(b), two plate-shaped resistivity measuring members 31 and 32 sandwiching the biological electrode 30 were horizontally placed, and a weight having a mass of 100g was placed on the resistivity measuring member 31 of vertically upper side. Considering the electrical resistance of the measuring electrodes 33 and 34 themselves, the constant current power supply 35 and the voltmeter 36 were arranged so that their respective electrical contacts with the measuring electrodes 33 and 34 were sufficiently separated from the sample placement positions 37 and 38. Further, the electrical contact of the constant current power supply 35 was set to a position 8mm away from one side edge of the measuring electrodes 33 and 34, and the electrical contact of the voltmeter 36 was set to a position further 8mm away in the same direction from the electrical contact of the constant current power supply 35. The size and the like of the measuring electrodes 33 and 34 were appropriately changed according to the size of the biological electrode 30 which is a sample.

Fig. 10 is a graph showing the measured results of the volume resistivity of the biological electrode (before applying the load) in Examples 1 and 2 and Comparative Example 1. In Fig. 10, the vertical axis represents the volume resistivity [Ω·cm]. As shown in Fig. 1, the volume resistivity of each of the biological electrode of Example 1 using the mixed silver powder 3 and the biological electrode of Example 2 using the mixed silver powder 4 was not significantly different from the volume resistivity of the biological electrode of Comparative Example 1 using the silver powder (pure silver particles) as it is, and it was found that the addition of fumed silica did not significantly affect the conductivity of the biological electrode immediately after producing. Note that, when the volume resistivity of the biological electrodes of Examples 1 and 2 was compared, it was found that the volume resistivity of the biological electrode of Example 2 using hydrophilic fumed silica was higher.

### <Strain resistance test>

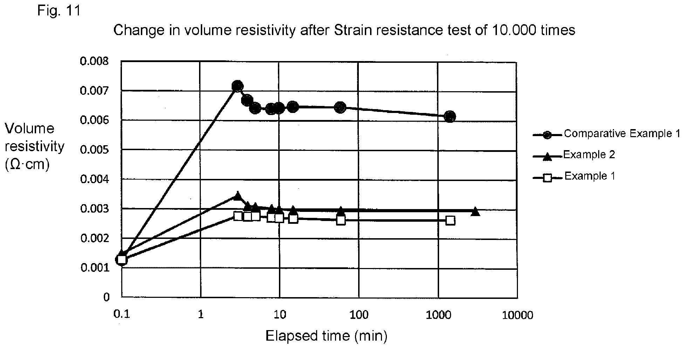

A load was applied to the biological electrodes in Examples 1 and 2 and Comparative Example 1 by the following method, and the volume resistivity [Ω·cm] of each biological electrode was measured over time immediately after the end of the load application. The volume resistivity of the biological electrode is measured as described above. A load of 1kgf was applied 10,000 times to each biological electrode formed to have a cylindrical shape with a diameter of 10mm at the end face and a length of 5mm in the axial direction. The load application cycle was 5,760 times/h. The measured results of the volume resistivity shown in Fig. 11.

Fig. 11 is a graph showing the measured results of the volume resistivity of the biological electrode (after applying the load) in Examples 1 and 2 and Comparative Example 1. In Fig. 11, the vertical axis represents the volume resistivity [Ω·cm], the horizontal axis represents the elapsed time [min] from the end of the load application. Fig. 11 is a semi-logarithmic graph where the horizontal axis is a logarithmic scale. Fig. 11 shows the change over time of the volume resistivity of each biological electrode from the end of the load application. In the graph shown in Fig. 11, the values of the volume resistivities [Ω·cm] of the respective biological electrode before applying the load are plotted at 0.1 minutes, which is the origin of the horizontal axis (see, for example, Fig. 10).

As shown in Fig. 11, the volume resistivity of the biological electrode in Comparative Example 1 using silver powder (pure silver particles) as it is, was increased to about 5 times as compared with that before the load application. The volume resistivity of the biological electrode in Comparative Example 1 was confirmed to have a tendency to gradually decrease thereafter, but when one day elapsed from the end of the load application, the volume resistivity was 4 times or more as compared with that before the load application, and the decrease in the volume resistivity thereafter became extremely slow, and the volume resistivity stagnated at a value substantially 4 times or more.

On the other hand, in the biological electrodes of Examples 1 and 2 in which a mixed silver powder composed of silver powder and fumed silica was used, an increase in the volume resistivity was suppressed to about 2 times as compared with that before applying the load. The rate at which the volume resistivity gradually decreased was smaller than that of Comparative Example 1. Therefore, it was found that the biological electrodes of Examples 1 and 2 exhibited a small change in resistivity with respect to strain. For this reason, for example, when these biological electrodes are used as an electroencephalogram measuring rubber electrode, it is possible to obtain an electrode which is hardly deteriorated in conductivity even in the pressing operation at the time of use or the repeated use of the electroencephalogram measuring rubber electrode. Further, as can be seen from the results of the sieve passage rate test of the mixed silver powder described above, the manufacturing method using the mixed silver powder 3 and 4 composed of silver powder and fumed silica had extremely good handling property during work.

### Industrial Applicability

As described above, the biological electrode obtained by the manufacturing method of the present invention can be suitably used particularly in the fields of medical measuring instruments, wearable information devices, game machines, brain machine interfaces, medical care, nursing care, welfare, automatic operation, electronic wiring, and the like.

### [Explanation of Reference Numerals]

1,2,3 Biological electrode
11 Conductive substrate
12 Conductive rubber body
13 Signal transmitting member
14 Living body
15 Covering wire
151 Core wire
152 Covering material
16 Flexible printed substrate
161 Base film
162 Conductive foil
17 Insulating layer
21 Disc
22 Protrusion
22a Protruding body
30 Biological electrode
31,32 Resistivity measuring member
33,34 Measuring electrode
35 Constant current power supply
36 Voltmeter
37,38 Sample placement position
221a Main body
222a Tip portion

## Claims

1. A method for manufacturing a biological electrode (1, 2, 3),
**characterized by**:
a step of adding fumed silica to an aggregated silver powder and mixing to obtain a mixed silver powder in which the silver powder and the fumed silica are dispersed, and
a step of forming a conductive rubber body (12) containing a silicone rubber and the mixed silver powder.

2. The method for manufacturing a biological electrode (1, 2, 3) according to claim 1, wherein hydrophobic fumed silica is used as the fumed silica.

3. The method for manufacturing a biological electrode (1, 2, 3) according to claim 1 or 2, wherein 1 to 5 parts by mass of the fumed silica is added to 100 parts by mass of the aggregated silver powder.

4. The method for manufacturing a biological electrode (1, 2, 3) according to any one of claims 1 to 3, wherein in the step of forming the conductive rubber body (12), the mixed silver powder is added to a room temperature-curable liquid silicone rubber to prepare a silver powder-containing paste, and the obtained silver powder-containing paste is cured to form the conductive rubber body (12).

5. The method for manufacturing a biological electrode (1, 2, 3) according to any one of claims 1 to 4, wherein in the step of forming the conductive rubber body (12), the conductive rubber body (12) having a layered shape is formed.

6. The method for manufacturing a biological electrode (1, 2, 3) according to any one of claims 1 to 4, wherein in the step of forming the conductive rubber body (12), the conductive rubber body (12) having a brushed shape is formed.

7. A rubber for a biological electrode (1, 2, 3), which includes at least silicone rubber, an aggregated silver powder, and a fumed silica, wherein 1 to 5 parts by mass of the fumed silica is contained per 100 parts by mass of the aggregated silver powder.

8. A rubber electrode made of the rubber for a biological electrode (1, 2, 3) according to claim 7.

## Patentansprüche

1. Verfahren zur Herstellung einer biologischen Elektrode (1, 2, 3),
**gekennzeichnet durch**:
einen Schritt des Hinzufügens von pyrogener Kieselsäure zu einem aggregierten Silberpulver und des Mischens, um ein gemischtes Silberpulver zu erhalten, in dem das Silberpulver und die pyrogene Kieselsäure dispergiert sind, und einen Schritt des Bildens eines leitfähigen Kautschukkörpers (12), der einen Silikonkautschuk und das gemischte Silberpulver enthält.

2. Verfahren zur Herstellung einer biologischen Elektrode (1, 2, 3) nach Anspruch 1, wobei hydrophobe pyrogene Kieselsäure als pyrogene Kieselsäure verwendet wird.

3. Verfahren zur Herstellung einer biologischen Elektrode (1, 2, 3) nach Anspruch 1 oder 2, wobei 1 bis 5 Masseteile der pyrogenen Kieselsäure zu 100 Masseteilen des aggregierten Silberpulvers gegeben werden.

4. Verfahren zur Herstellung einer biologischen Elektrode (1, 2, 3) nach einem der Ansprüche 1 bis 3, wobei in dem Schritt der Bildung des leitfähigen Kautschukkörpers (12) das gemischte Silberpulver zu einem bei Raumtemperatur härtbaren flüssigen Silikonkautschuk hinzugefügt wird, um eine silberpulverhaltige Paste herzustellen, und die erhaltene silberpulverhaltige Paste gehärtet wird, um den leitfähigen Kautschukkörper (12) zu bilden.

5. Verfahren zur Herstellung einer biologischen Elektrode (1, 2, 3) nach einem der Ansprüche 1 bis 4, wobei in dem Schritt der Bildung des leitfähigen Kautschukkörpers (12) der leitfähige Kautschukkörper (12) mit einer geschichteten Form gebildet wird.

6. Verfahren zur Herstellung einer biologischen Elektrode (1, 2, 3) nach einem der Ansprüche 1 bis 4, wobei in dem Schritt der Bildung des leitfähigen Kautschukkörpers (12) der leitfähige Kautschukkörper (12) mit einer gebürsteten Form gebildet wird.

7. Kautschuk für eine biologische Elektrode (1, 2, 3), der mindestens Silikonkautschuk, ein aggregiertes Silberpulver und pyrogene Kieselsäure enthält, wobei 1 bis 5 Masseteile der pyrogenen Kieselsäure pro 100 Masseteile des aggregierten Silberpulvers enthalten sind.

8. Kautschukelektrode, die aus dem Kautschuk für eine biologische Elektrode (1, 2, 3) nach Anspruch 7 besteht.

## Revendications

1. Procédé de fabrication d'une électrode biologique (1, 2, 3),
**caractérisé par** :
une étape d'ajout de silice sublimée à une poudre d'argent agrégée, et de mélange pour obtenir une poudre d'argent mélangée où la poudre d'argent et la silice sublimée sont dispersées, et
une étape de formation d'un corps en caoutchouc conducteur (12) contenant un caoutchouc de silicone et la poudre d'argent mélangée.

2. Procédé de fabrication d'une électrode biologique (1, 2, 3) selon la revendication 1, où de la silice sublimée hydrophobe est utilisée en tant que silice sublimée.

3. Procédé de fabrication d'une électrode biologique (1, 2, 3) selon la revendication 1 ou la revendication 2, où 1 à 5 parties en masse de la silice sublimée sont ajoutées à 100 parties en masse de la poudre d'argent agrégée.

4. Procédé de fabrication d'une électrode biologique (1, 2, 3) selon l'une des revendications 1 à 3, où, lors de l'étape de formation du corps en caoutchouc conducteur (12), la poudre d'argent agrégée est ajoutée à un caoutchouc de silicone liquide durcissant à température ambiante pour préparer une pâte contenant de la poudre d'argent, et où la pâte contenant de la poudre d'argent obtenue est durcie pour former le corps en caoutchouc conducteur (12).

5. Procédé de fabrication d'une électrode biologique (1, 2, 3) selon l'une des revendications 1 à 4, où, lors de l'étape de formation du corps en caoutchouc conducteur (12), le corps en caoutchouc conducteur (12) ayant une forme stratifiée est formé.

6. Procédé de fabrication d'une électrode biologique (1, 2, 3) selon l'une des revendications 1 à 4, où, lors de l'étape de formation du corps en caoutchouc conducteur (12), le corps en caoutchouc conducteur (12) ayant une forme en brosse est formé.

7. Caoutchouc pour électrode biologique (1, 2, 3), contenant au moins un caoutchouc de silicone, une poudre d'argent agrégée et une silice sublimée, où 1 à 5 parties en masse de la silice sublimée sont contenues pour 100 parties en masse de poudre d'argent agrégée.

8. Electrode en caoutchouc pour électrode biologique (1,2,3) selon la revendication 7.
